Europäisches Patentamt

European Patent Office

Office européen des brevets

(11) Publication number: **0 145 469**
**B1**

(19)

(12) # EUROPEAN PATENT SPECIFICATION

(45) Date of publication of patent specification: **27.01.88**

(51) Int. Cl.⁴: **C 07 C 51/25,** C 07 C 57/04, C 07 C 57/08 // B01J23/44

(21) Application number: **84308538.2**

(22) Date of filing: **07.12.84**

(54) **Increased selectivities in the oxidation of olefins to alpha, beta-unsaturated carboxylic acids.**

(30) Priority: **07.12.83 US 559056**
**29.10.84 US 664564**

(43) Date of publication of application:
**19.06.85 Bulletin 85/25**

(45) Publication of the grant of the patent:
**27.01.88 Bulletin 88/04**

(84) Designated Contracting States:
**BE DE FR GB IT NL**

(56) References cited:
**CH-A- 376 485**
**GB-A-1 361 811**
**GB-A-1 535 584**

(73) Proprietor: **Sun Refining and Marketing Company**
**1801 Market Street**
**Philadelphia, PA 19103 (US)**

(72) Inventor: **Lyons, James E.**
**211 Cooper Drive**
**Wallingford, PA 19086 (US)**

(74) Representative: **Lewin, John Harvey et al**
**Elkington and Fife High Holborn House 52/54**
**High Holborn**
**London WC1V 6SH (GB)**

**Description**

Background of the invention
Field of the invention

This invention relates to a process for the oxidation of olefins to form α,β-unsaturated carboxylic acids. More particularly, this invention relates to an improved method for the oxidation of propylene to acrylic acid in the presence of a novel olefin-activated palladium catalyst and a free radical inhibitor.

In a like manner, isobutylene and butene-1 may be oxidized respectively to methacrylic acid and crotonic acid.

Description of the prior art

The oxidation of propylene to acrylic acid in one step employing a palladium metal catalyst supported on carbon black is described in U.S. Patent 3,624,147. However, this process is characterized by yields of 60% or less, based on the amount of propylene converted, operating temperatures generally in excess of 90°C, and elevated pressures. Moreover, substantial amounts of $CO_2$ are reported as undesired by-products, as well as low reaction rates.

A similar process is reported in *J. Catal.*, 173 (1972) by Sieyama et al., in which palladium black and palladium-activated charcoal were employed for converting propylene to acrylic acid. However, only a stoichiometric, non-catalytic conversion, based on the palladium metal, is taught, thus providing an even less effective method than in the above U.S. Patent.

In addition, several patents describe conventional methods for the preparation of supported palladium metal catalysts by the reduction of, for example, palladium salts using such reducing agents as hydrogen, lower alcohols, hydrazine, or various olefins. See, for example, U.S. Patents 3,275,680 to Holzrichter, or 4,435,598 to Hinnenkamp which teach reducing palladium salts with hydrogen or hydrazine. U.S. Patent 4,016,200 to Onoda likewise teaches that a palladium compound can be reduced to palladium metal, using formalin, hydrazine, hydrogen, methanol or olefins such as ethylene, propylene, or butene as reducing agents. Similarly, U.S. Patent 3,970,713 to Scharfe teaches the reduction of palladium and other metal salts to a metal catalyst, again using hydrogen, alcohols, olefins, etc., as reducing agents. However, none of these references teaches the preparation of a highly activated species of palladium metal which has been activated with an olefin under unique time and temperature conditions, or that such a catalyst is surprisingly effectively in a process for the oxidation of olefins to α,β-unsaturated acids under more moderate operating conditions than have heretofore been possible. Finally, F. R. Hartley, "The Chemistry of Platinum and Palladium", Wiley and Sons, pp. 386—390, and 412—417 (1973) disclose the formation of a complex of ethylene with a palladium chloride to provide a palladium$^{+2}$ metal catalyst. However, as will be described below, the use of ethylene, or chlorides, and the formation of palladium$^{+2}$ metal catalysts have been found to deactivate the catalyst of this invention for purposes of forming the desired products claimed herein.

In U.S. application (Docket No. 83-046 CIP-1), referred to above, which is incorporated herein by reference in its entirety, and filed simultaneously herewith, there is described an improved method for the oxidation of propylene directly to acrylic acid with air or oxygen in an aqueous medium under moderate reaction conditions employing a supported palladium catalyst which has been pretreated by activating it with a $C_3$—$C_6$ olefin, preferably propylene, at temperatures of at least about 60°C for at least about 10 minutes. The oxidation reaction is then continued in the presence of the pretreated catalyst at temperatures of at least about 25°C, whereby acrylic acid is recovered at high rates and selectivities as contrasted with the above prior art.

It is thus an object of the present invention to provide a further improvement of the afore-described olefin oxidation process, using certain free radical inhibitors, as set forth hereinbelow.

It is a further object of this invention to oxidize isobutylene to methacrylic acid and butene-1 to crotonic acid in the same manner.

Summary of the invention

In accordance with the present invention, it has now been found that the selectivities obtained during the oxidation of olefins such as propylene to form α,β-unsaturated carboxylic acids such as acrylic acid may be further enhanced over those obtained by the use of an olefin-activated palladium catalyst supported on carbon or alumina, as described in copending application (Docket No. 83-046 CIP-1) and below, when the oxidation is carried out in the added presence of free radical inhibitors. By carrying out the aforesaid oxidation in the presence of these inhibitors, it has been discovered that selectivities to acrylic acid are obtained in the range of up to about 92—93%, i.e., an enhancement of about 5—12% over the use of the activated catalyst alone. Thus, by using this expedient, the yields of unwanted by-product may be surprisingly diminished by a factor of two or more.

This same catalyst system, in combination with a radical inhibitor, is likewise effective in oxidizing isobutylene to methacrylic acid, and butene-1 to crotonic acid.

Thus, olefins having from three to six carbon atoms may be employed in this invention.

The present invention provides a process for the preparation of α,β-unsaturated carboxylic acids which comprises contacting a supporting palladium metal catalyst with a $C_3$—$C_6$ olefin admixed with air or

2

oxygen in a liquid medium, thereby oxidizing said olefin to the corresponding carboxylic acid, characterised in that the catalyst is first activated by contacting it with said $C_3$—$C_6$ olefin in said liquid medium at a temperature of at least about 60°C for at least about 10 minutes in the substantial absence of oxygen, and the oxidation step is carried out in the presence of a free radical inhibitor.

The invention also provides a process for the preparation of α,β-unsaturated carboxylic acids which comprises contacting a supported palladium metal catalyst with a $C_3$—$C_6$ olefin admixed with air or oxygen in a liquid medium, thereby oxidizing said olefin to the corresponding carboxylic acid, characterised in that the catalyst is first activated by contacting it with a different $C_3$—$C_6$ olefin in said liquid medium at a temperature of at least about 60°C for at least about 10 minutes in the substantial absence of oxygen, and the oxidation step is carried out in the presence of a free radical inhibitor.

Description of the preferred embodiments

As described above, the present invention is directed to a further improvement in a novel process for the oxidation of certain olefins, principally propylene, to their corresponding acids in the presence of a pretreated supported palladium catalyst, said improvement comprising carrying out the oxidation with air or oxygen in an aqueous medium in the presence of a free radical inhibitor such as butylated hydroxy toluene (BHT).

The general method of oxidizing propylene to acrylic acid is adequately described in the prior art and need not be described herein in detail. Suffice it to say that utilizing the catalyst prepared by the novel methods discussed in detail below in combination with applicant's claimed free radical inhibitor, the oxidation reaction of propylene to acrylic acid can then be uniquely carried out at temperatures in the range of from about 25 to 125°C, and at pressures of 1 to 100 atm. Preferably, temperatures of from 25 to 85°C and pressures of from 1 to 10 atm may be employed, as contrasted with the much more rigorous conditions employed in U.S. 3,624,147. As a consequence of this combined catalyst and inhibitor, selectivities, and thus yields, are significantly increased over the use of the catalyst alone, as shown in the examples below.

In one preferred embodiment of this process, in order to increase the reaction rate and at the same time reduce the reactor volume, it has been found to be advantageous that the reaction be carried out in a trickle bed reactor in which the liquid reaction medium is allowed to pass downward over a fixed catalyst bed and the acrylic acid product recovered at the bottom. Alternatively, the oxidation reaction can be carried out using an ebulating bed of catalyst while circulating gases and solvent.

The starting material from which the catalyst of this invention is prepared may be any finely divided palladium in the metallic state, on a support such as carbon or, less preferred, alumina, as for example a commercially available 5%, 10%, or 20% palladium on carbon available from standard catalyst manufacturers such as Engelhard Industries or Johnson Mathey, Inc. By the terms "palladium metal catalyst" or "palladium in the metallic state" is meant those palladium catalysts which have been prepared from their salts by known reduction means either commercially or as shown, for example, by Scharfe et al, U.S. Patent 3,970,713, or Holzrichter et al, U.S. Patent 3,275,680, but which have subsequently been exposed to the atmosphere in normal process procedures. While applicants do not wish to be bound by any particular theories, it is believed that in the normal course of handling and using the reduced catalysts of the prior art subsequent to reduction of the palladium, a certain proportion of the palladium surface species, by virtue of exposure to the atmosphere, becomes oxidized. It is this air-exposed palladium catalyst which is now being employed as the starting material in the preparation of applicants' novel olefin-activated catalyst. (By "surface species", as recognized by those skilled in the catalyst art, is meant any species of palladium found at the surface of the catalyst per se.).

Again, while applicants do not wish to be bound by any particular theory, it is believed that when this partly oxidized palladium surface, as described above, is contacted with propylene in accordance with applicant's invention, it is first converted to highly active palladium metal sites having zero valence, and it is with these sites that the propylene then forms the novel surface-active species which is the activated catalyst of this invention.

As evidence that the commercially-reduced palladium, for example, has been reoxidized under normal handling and exposure to air, it has been found that in the course of preparing the novel activated catalyst of this invention, starting, e.g. with a commercially reduced palladium metal catalyst under oxygen-free conditions as described below, two parts propylene employed in activating the catalyst result in the formation of one part acetone and one part active catalyst species.

In preparing the activated oxidation catalyst used in this invention by treating a carbon-or alumina-supported palladium metal catalyst as defined above with propylene or like olefins, it is essential that this activation treatment be carried out at temperatures of at least about 60°C, up to 150°C, preferably about 65 to 95°C, for a period of at least about 10 minutes to about 120 minutes, preferably at least about 30 to 60 minutes, under oxygen-free conditions as described below. This is generally carried out at pressures of at least about 1 atmosphere, up to about 100 atmospheres of propylene, although about 2—20 atmospheres is preferred. When these catalysts are thus activated, palladium-on-carbon, for example, which was otherwise far less reactive at temperatures below about 60°C for purposes of oxidizing propylene is now surprisingly active at temperatures of about 25°C or above. Moreover, as aforestated, the selectivities to acrylic acid are significantly improved by this treatment which is further enhanced by the use of applicant's defined inhibitors. Thus, by the term "activated palladium metal catalyst" is meant, for purposes of this invention, a catalyst prepared in accordance with the above method, and which can oxidize propylene to acrylic acid more rapidly and at lower temperatures than known supported palladium catalysts.

During the preparation of the catalyst, as stated above, it is necessary for purposes of deriving maximum activity from the catalyst that the activation be carried out in the substantial absence of oxygen, and preferably under essentially oxygen-free conditions. While the presence of small amounts of oxygen, to an extent which can be readily determined by those skilled in the art, can still result in a catalyst which performs under somewhat more mild conditions than the commercial catalysts described above, the full benefits of the present invention are derived from activating the catalyst under conditions which are as oxygen-free as can be obtained, at least within the standards of commercial feasibility.

These oxygen-free conditions can be achieved by known means, for example by using deaerated water or solvent, and pure olefin gas, during the activation of the catalyst. Deaeration can be readily achieved by placing the liquid under vacuum until it boils, or by bubbling the desired olefin through the liquid for a period of time until no more oxygen is displaced. The pure olefin can be obtained commercially in various grades such as chemical purity grade, research purity grade, or polymer grade, the latter two being preferred because of their higher purity of over about 99.7%. (The latter two are available, for example from Matheson, Division of Searle Medical Products, and Sun Co., respectively).

Once applicants' catalyst is formed, it is preferable that at least a slight excess of olefin be present at all times to prevent any deactivation, and that desirably during the oxidation step, oxygen in the reactor be maintained in no greater than the stoichiometric amounts needed for the oxidation of the olefin to acrylic acid. It will also be understood that in preparing the catalyst of this invention, the presence of those metals or metal salts which might poison or alter the catalyst should be avoided, for example iron, manganese, copper and rhodium salts; chlorides, benzoquinone, the oxidized form of heteropoly acids, as well as any other agents which would oxidize palladium to palladium$^{+2}$. Other such deleterious materials can be routinely determined by those skilled in the art. For example, in addition, it has been found that such materials as amines, hydrazine, and ethylene should be avoided as deleterious when preparing and using the catalyst of this invention. Moreover, it has been found that attempts to use hydrogen to prepare this catalyst may result in explosions when the catalyst is exposed to $O_2$-propylene mixtures, and should also be avoided.

While the catalyst of the invention may be prepared separately and maintained in an active state if kept in an oxygen-free atmosphere, more conveniently the preparation is carried out in the same reactor used for the propylene oxidation. This may conveniently be achieved, for example by adding a commercially available finely divided palladium on activated carbon to an aqueous medium in a sealed reactor, flushing the system with propylene gas, and then heating the mixture under propylene pressure until the desired temperature for preparation of the catalyst is reached, at which time the mixture is stirred for at least 30 minutes at that temperature, again, in the absence of oxygen, and desirably in the presence of a slight excess of olefin.

After the preparation of the catalyst, the propylene may be replaced by a mixture of propylene and oxygen, desirably with oxygen being present in approximately stoichiometric amounts to avoid deactivation of the catalyst, the free radical inhibitor added to the liquid medium, if it is not already present, and the oxidation reaction carried out at pressures of from about 1 to 10 atmospheres. The pressure may be maintained by the further addition of the gas mixture from time to time until the desired propylene conversion is achieved. Air may be used in place of oxygen, in which case the amount of propylene must be adjusted proportionately.

While the activating agent for the catalyst is preferably propylene, if desired there may instead be employed other light olefins having an allylic hydrogen and containing from 3—6 carbon atoms, preferably those corresponding to the olefins to be oxidized. Most preferred, in addition to propylene, are butene-1, butene-2 or isobutylene.

The olefin-activated catalyst maintains its activity over long periods of time as long as at least small amounts of an acceptable olefin are present. Thus, it has been found beneficial to run the reaction by constantly sparging the propylene/oxygen or air reaction mixture through the aqueous solution. In this way, the propylene is kept in excess and the catalyst remains highly active, thereby maintaining high selectivities and other advantages noted above.

When carrying out the oxidation in a batch-wise manner the ratio of catalyst to reaction medium is desirably in the range of about 0.05—5.0 gram atoms of palladium per liter of reactant, and preferably about 0.1—1.0 gram atoms. In a continuous process utilizing, e.g., a fixed bed reactor, the reaction can be conducted effectively by varying the volume of reactants and contact time with the catalyst in a generally known manner to achieve the high yields and selectivities disclosed herein.

In general, many types of free radical inhibitors known to those skilled in the art may be employed in the process of this invention. Good selectivities for acrylic acid, mostly by reduction of acetone formation, make the use of these inhibitors a superior process for the oxidation of propylene to acrylic acid. In the absence of these inhibitors, selectivities are typically in the range of about 80—85%, whereas the introduction of e.g., BHT, as shown by Table I, can surprisingly increase selectivities to up to about 92—93%. The free radical inhibitor may be added to the liquid medium at any point prior to oxidation, i.e. either before or after activation.

In addition to butylated hydroxytoluene, (BHT), there may also be employed such compounds as 2,2'-methylenebis(4-methyl-6-*tert*-butylphenol), hydroquinone, sulfur-containing metal chelates such as zinc dithiocarbamates and zinc dithiophosphates, and the like.

The amount of free radical inhibitor employed is generally in the range of from about 0.001—1.0 grams per liter of aqueous medium, and preferably about 0.01—0.5 grams per liter.

The following examples are by way of illustration of the invention.

Examples 1—6

In the following examples, 1—6, a number of reactions were run in accordance with the following general procedures:

One gram of 10% palladium on carbon (Engelhard Industries) was added to an 85 ml Fisher-Porter aerosol tube. Then 30 ml of deaerated distilled water was added and the Fisher-Porter tube was fitted to a pressure manifold. BHT, in the amount listed in the Table, was then added.

The mixture was flushed to 50 psi three times with pure propylene gas (research purity grade). It was then heated with stirring under 50 psi of this pure propylene until it reached the desired activation temperature where the mixture was stirred for 30 minutes. The stirred mixture was then brought to the desired reaction temperature and the propylene was replaced with a gas mixture having the composition: 60% $O_2$/40% pure $C_3H_6$ to a total pressure of 100 psig. The reaction proceeded immediately in most cases and the pressure dropped. When the total pressure reached 80 psig the $O_2$/$C_3H_6$ gas mixture was admitted to bring the total pressure to 100 psig. This was repeated as often as necessary during the course of the run. After the determined reaction time the mixture was cooled, the gas captured and analyzed and the mixture filtered. The catalyst was washed with both organic and aqueous solutions to remove acrylic acid held on the surface. The filtrates were analyzed by standardized GC to determine the product composition.

The results, together with certain variations in the reaction conditions, are shown in Table 1 below. In certain of the examples, where indicated, the catalyst were first washed with a base to remove any trace quantities of chloride ion remaining from their commercial preparation.

TABLE 1

Effect of a free radical inhibitor on rate and selectivity of catalytic propylene oxidation

| Example | Catalyst description | BHT added, GM | Activation Temp., C | Reaction Temp., C | Reaction time, hrs. | Gas Up-take psi | Liquid analysis gms./liter-/wt.% | | | | Acrylic acid | CO₂ mmoles | Acrylic acid production | | |
|---|---|---|---|---|---|---|---|---|---|---|---|---|---|---|---|
| | | | | | | | Acetal-dehyde | Acetone | Acrolein | Acetic acid | | | Yield g/l | Select-ivity % | Run rate psi/hr. |
| 1 | 10% Pd-C, Englehard | 0.0 | 65 | 65 | 4 | 149 | 0.88 2.02 | 2.77 6.37 | 0.70 1.61 | 1.32 3.03 | 37.83 86.97 | 2.5 | 37.8 | 87.0 | 37.3 |
| 2 | 10% Pd-C, Englehard | 0.0 | 65 | 65 | 4 | 142 | 0.42 1.17 | 2.35 6.57 | 0.31 0.87 | 1.39 3.89 | 31.31 87.53 | 2.3 | 31.3 | 87.5 | 35.5 |
| 3 | 10% Pd-C, Englehard | 0.1 | 65 | 65 | 4 | 149 | 0.48 0.99 | 0.88 1.82 | 0.24 0.50 | 1.94 4.01 | 44.84 92.68 | 2.45 | 44.8 | 92.7 | 37.3 |
| 4 | 10% Pd-C, Englehard Base treat type 1 | 0.0 | 65 | 65 | 4 | 149[a] | 0.50 2.20 | 2.04 8.97 | 0.24 1.05 | 1.67 7.34 | 18.3 80.43 | 1.8 | 18.3 | 80.4 | 37.3[a] |
| 5 | 10% Pd-C, Englehard Based treat type 1 | 0.1 | 65 | 65 | 4 | 149[a] | 0.47 1.85 | 1.02 4.01 | 0.23 2.90 | 1.56 6.13 | 22.18 87.18 | 2.6 | 22.2 | 87.2 | 37.3[a] |
| 6 | 10% Pd-C, Englehard Based treat type 1 | 0.1 | 65 | 65 | 4 | 161[a] | 0.85 3.23 | 1.44 5.57 | 0.23 0.89 | 1.31 5.07 | 22.00 85.17 | 2.2 | 22.0 | 85.2 | 40.3[a] |

[a] Reactor developed a slow leak so uptakes (and therefore rates of gas uptake) are higher than actual.

Example 7

When the reaction is carried out in accordance with the procedures of Example 3, but substituting isobytylene for propylene, there is obtained methacrylic acid as a major product.

Example 8

When the reaction is carried out in accordance with the procedure of Example 3, but substituting butene-1 for propylene, there is obtained crotonic acid as a major product.

**Claims**

1. A process for the preparation of α,β-unsaturated carboxylic acids which comprises contacting a supported palladium metal catalyst with a $C_3$—$C_6$ olefin admixed with air or oxygen in a liquid medium, thereby oxidizing said olefin to the corresponding carboxylic acid, characterised in that the catalyst is first activated by contacting it with said $C_3$—$C_6$ olefin in said liquid medium at a temperature of at least about 60°C for at least about 10 minutes in the substantial absence of oxygen, and the oxidation step is carried out in the presence of a free radical inhibitor.

2. A process for the preparation of α,β-unsaturated carboxylic acids which comprises contacting a supported palladium metal catalyst with a $C_3$—$C_6$ olefin admixed with air or oxygen in a liquid medium, thereby oxidizing said olefin to the corresponding carboxylic acid, characterised in that the catalyst is first activated by contacting it with a different $C_3$—$C_6$ olefin in said liquid medium at a temperature of at least about 60°C for at least about 10 minutes in the substantial absence of oxygen, and the oxidation step is carried out in the presence of a free radical inhibitor.

3. A process according to Claim 1 or 2, wherein the catalyst is activated in the essential absence of oxygen.

4. A process according to any of Claims 1 to 3, wherein the catalyst is maintained in its activated state by continuous contact with said olefin.

5. A process according to any of Claims 1 to 3, wherein the catalyst is maintained in its activated state by continuous contact with said olefin being oxidized.

6. A process according to any of Claims 1 to 5, wherein the catalyst is activated with said olefin under a pressure of from 1 to 100 atmospheres of said olefin.

7. A process according to any of Claims 1 to 6, wherein the catalyst is activated with said olefin at a temperature of from 60°C to 150°C for 10 to 120 minutes.

8. A process according to any of Claims 1 to 7, wherein the olefin is propylene and the carboxylic acid is acrylic acid.

9. A process according to any of Claims 1 to 7, wherein the olefin is isobutylene and the carboxylic acid is methacrylic acid.

10. A process according to any of Claims 1 to 7, wherein the olefin is butene-1 and the carboxylic acid is crotonic acid.

11. A process according to any of Claims 1 to 10, wherein the oxidation is carried out with stoichiometric amounts of olefin and oxygen needed to produce said α,β-unsaturated carboxylic acid.

12. A process according to any of Claims 1 ho 11, wherein the support for the palladium metal is carbon or alumina.

13. A process according to any of Claims 1 to 12, wherein the oxidation is carried out at a temperature of at least 25°C.

14. A process according to any of Claims 1 to 13, wherein the free radical inhibitor is butylated hydroxytoluene.

15. A process according to any of Claims 1 to 13, wherein the free radical inhibitor is 2,2'-methylenebis(4-methyl-6-tert-butylphenol).

16. A process according to any of Claims 1 to 15, wherein the amount of free radical inhibitor employed is from 0.001—1.0 grams per litre of aqueous medium.

**Patentansprüche**

1. Verfahren zur Herstellung von α,β-ungesättigten Carbonsäuren, bei dem ein Palladiummetall-Trägerkatalysator mit einem $C_3$—$C_6$-Olefin, dem Luft oder Sauerstoff zugemischt ist, in einem flüssigen Medium in Kontakt gebracht wird, wobei das Olefin zur entsprechenden Carbonsäure oxidiert wird, dadurch gekennzeichnet, daß der Katalysator zuerst aktiviert wird, indem er mit dem $C_3$—$C_6$-Olefin in dem flüssigen Medium bei einer Temperatur von mindestens etwa 60°C während mindestens etwa 10 Minuten im wesentlichen in Abwesenheit von Sauerstoff in Kontakt gebracht wird, und der Oxidationsschritt in Anwesenheit eines Inhibitors für freie Radikale durchgeführt wird.

2. Verfahren zur Herstellung von α,β-ungesättigten Carbonsäuren, bei dem ein Palladiummetall-Trägerkatalysator mit einem $C_3$—$C_6$-Olefin, dem Luft oder Sauerstoff zugemischt ist, in einem flüssigen Medium in Kontakt gebracht wird, wobei das Olefin zur entsprechenden Carbonsäure oxidiert wird, dadurch gekennzeichnet, daß der Katalysator zuerst aktiviert wird, indem er mit einem unterschiedlichen $C_3$—$C_6$-Olefin in dem flüssigen Medium bei einer Temperatur von mindestens etwa 60°C während

**0 145 469**

mindestens etwa 10 Minuten im wesentlichen in Abwesenheit von Sauerstoff in Kontakt gebracht wird, und der Oxidationsschritt in Anwesenheit eines Inhibitors für freie Radikale durchgeführt wird.

3. Verfahren gemäß Anspruch 1 oder 2, bei dem der Katalysator im wesentlichen in Abwesenheit von Sauerstoff aktiviert wird.

4. Verfahren gemäß einem der Ansprüche 1 bis 3, bei dem der Katalysator durch fortwährenden Kontakt mit dem Olefin in seinem aktivierten Zustand gehalten wird.

5. Verfahren gemäß einem der Ansprüche 1 bis 3, bei dem der Katalysator durch fortwährenden Kontakt mit dem Olefin, das oxidiert wird, in seinem aktivierten Zustand gehalten wird.

6. Verfahren gemäß einem der Ansprüche 1 bis 5, bei dem der Katalysator mit dem Olefin unter einem Druck von 1 bis 100 bar (Atmosphären) aktiviert wird.

7. Verfahren gemäß einem der Ansprüche 1 bis 6, bei dem der Katalysator bei einer Temperatur von 60 bis 150°C während 10 bis 120 Minuten aktiviert wird.

8. Verfahren gemäß einem der Ansprüche 1 bis 7, bei dem das Olefin Propylen und die Carbonsäure Acrylsäure ist.

9. Verfahren gemäß einem der Ansprüche 1 bis 7, bei dem das Olefin Isobutylen und die Carbonsäure Methacrylsäure ist.

10. Verfahren gemäß einem der Ansprüche 1 bis 7, bei dem das Olefin Buten-1 und die Carbonsäure Crotonsäure ist.

11. Verfahren gemäß einem der Ansprüche 1 bis 10, bei dem die Oxidation mit stöchiometrischen Mengen an Olefin und Sauerstoff, welche zur Herstellung der $\alpha,\beta$-ungesättigten Carbonsäure benötigt werden, durchgeführt wird.

12. Verfahren gemäß einem der Ansprüche 1 bis 11, bei dem der Träger für das Palladiummetall Kohlenstoff oder ein Aluminiumoxid ist.

13. Verfahren gemäß einem der Ansprüche 1 bis 12, bei dem die Oxidation bei einer Temperatur von mindestens 25°C durchgeführt wird.

14. Verfahren gemäß einem der Ansprüche 1 bis 13, bei dem der Inhibitor für freie Radikale butyliertes Hydroxytoluol ist.

15. Verfahren gemäß einem der Ansprüche 1 bis 13, bei dem der Inhibitor für freie Radikale 2,2'-Methylenbis(4-methyl-6-tert.-butylphenol) ist.

16. Verfahren gemäß einem der Ansprüche 1 bis 15, bei dem die Menge des verwendeten Inhibitors für freie Radikale 0,001 bis 1 g/l des wässrigen Mediums beträgt.

## Revendications

1. Procédé pour la préparation d'acides carboxyliques $\alpha,\beta$-insaturés qui comprend la mise en contact d'un catalyseur métallique au palladium sur support, avec une oléfine en $C_3$ à $C_6$ mélangée avec de l'air ou de l'oxygène, dans un milieu liquide, aboutissant à l'oxydation de cette oléfine en acide correspondant, caractérisé en ce que le catalyseur est d'abord activé par mise en contact avec l'oléfine en $C_3$ à $C_6$ dans le milieu liquide, à une température d'au moins 60°C environ pendant au moins 10 minutes environ, en l'absence marquée d'oxygène, et en ce que le stade d'oxydation est réalisé en présence d'un inhibteur de radical libre.

2. Procédé pour la préparation d'acides carboxyliques $\alpha,\beta$-insaturés, qui comprend la mise en contact d'un catalyseur métallique au palladium sur support, avec une oléfine en $C_3$ à $C_6$ mélangée avec de l'air ou de l'oxygène, dans un milieu liquide, aboutissant à l'oxydation de cette oléfine en acide correspondant, caractérisé en ce que le catalyseur est d'abord activé par mise en contact avec une oléfine en $C_3$ à $C_6$ différente, dans le milieu liquide, à une température au moins égale à 60°C environ pendant au moins 10 minutes environ, en l'absence marquée d'oxygène, et en ce que le stade d'oxydation est réalisé en présence d'un inhibiteur de radical libre.

3. Procédé selon la revendication 1 ou 2, dans lequel le catalyseur est activé pratiquement en absence d'oxygène.

4. Procédé selon une des revendications 1 à 3, dans lequel le catalyseur est maintenu à son état activé par contact continu avec l'oléfine.

5. Procédé selon une des revendications 1 à 3, dans lequel le catalyseur est maintenu à son état activé par contact continu avec l'oléfine à oxyder.

6. Procédé selon une des revendications 1 à 5, dans lequel le catalyseur est activé avec l'oléfine sous une pression de 1 à 100 atmosphères de-la-dite oléfine.

7. Procédé selon une des revendications 1 à 6, dans lequel le catalyseur est activé avec l'oléfine à une température de 60° à 150°C, pendant 10 à 120 minutes.

8. Procédé selon une des revendications 1 à 7, dans lequel l'oléfine est du propylène, et l'acide carboxylique l'acide acrylique.

9. Procédé selon une des revendications 1 à 7, dans lequel l'oléfine est de l'isobutylène et l'acide carboxylique l'acide méthacrylique.

10. Procédé selon une des revendications 1 à 7, dans lequel l'oléfine est le butène-1, et l'acide carboxylique l'acide crotonique.

11. Procédé selon une des revendications 1 à 10, dans lequel l'oxydation est réalisée avec les quantités stoechiométriques d'oléfine et d'oxygène nécessaires pour produire l'acide carboxylique α,β-insaturé.

12. Procédé selon une des revendications 1 à 11, dans lequel le support du palladium métallique est du carbone ou de l'alumine.

13. Procédé selon une des revendications 1 à 12, dans lequel l'oxydation est réalisée à une température supérieure à 25°C.

14. Procédé selon une des revendications 1 à 13, dans lequel l'inhibiteur de radical libre est de l'hydroxytoluène butylé.

15. Procédé selon une des revendications 1 à 13, dans lequel l'inhibiteur de radical libre est le méthylène-2,2'bis-(méthyl-4 tert-butyl-6 phénol).

16. Procédé selon une des revendications 1 à 15, dans lequel la quantité d'inhibiteur de radical libre utilisée, est de 0,001 à 1 g/litre de milieu aqueux.